# EUROPÄISCHE PATENTSCHRIFT

(11) **EP 1 902 042 B1**
(45) Veröffentlichungstag und Bekanntmachung des Hinweises auf die Patenterteilung: **16.12.2009**
(21) Anmeldenummer: 06818209.6
(22) Anmeldetag: 24.06.2006
(51) Int. Cl.: C07D 275/02

(54) **VERFAHREN ZUR HERSTELLUNG VON 3,4-DICHLOR-ISOTHIAZOLCARBONSÄURE**
PROCESS FOR PREPARING 3,4-DICHLOROISOTHIAZOLECARBOXYLIC ACID
PROCEDE DE PREPARATION D'ACIDE 3,4-DICHLORO-ISOTHIAZOLCARBOXYLIQUE

(30) Priorität: 05.07.2005 DE 102005031348
(43) Veröffentlichungstag der Anmeldung: 26.03.2008
(73) Patentinhaber: Bayer CropScience Aktiengesellschaft, 40789 Monheim (DE)
(72) Erfinder: HIMMLER, Thomas, 51519 Odenthal (DE)
(86) Internationale Anmeldenummer: PCT/EP2006/006119
(87) Internationale Veröffentlichungsnummer: WO 2007/025585

(56) Entgegenhaltungen:
- US-A- 3 341 547
- US-A- 3 393 992
- US-A- 5 240 951

## Beschreibung

Die Erfindung betrifft ein Verfahren zur Herstellung und Isolierung von 3,4-Dichlor-isothiazol-carbonsäure.

3,4-Dichlor-isothiazol-carbonsäure der Formel (I) ist eine bekannte Verbindung. Ihre Herstellung wird beispielsweise in US 3,341,547 und US 3,393,992 beschrieben. Dabei werden in einem ersten Schritt Natriumcyanid, Schwefelkohlenstoff und Chlor in einem Lösungsmittel, beispielsweise N,N-Dimethyl-formamid (DMF), umgesetzt, wodurch 3,4-Dichlor-isothiazol-carbonitril der Formel (II) erhalten wird. Anschließend wird in einem zweiten Reaktionsschritt das 3,4-Dichlor-isothiazol-carbonitril durch Erhitzen in Natronlauge zur 3,4-Dichlor-isothiazol-carbonsäure verseift:

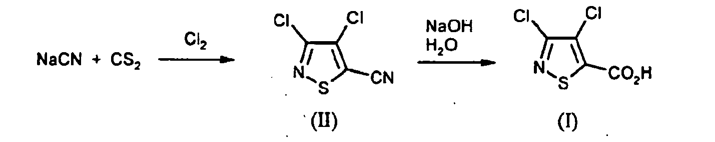

Die Verseifung des Nitrils der Formel (II) kann grundsätzlich auch sauer durchgeführt werden. Dies hat jedoch den Nachteil, dass sowohl das Nitril als auch die Säure unter solchen Bedingungen sublimieren, was im technischen Maßstab zu Problemen führt.

Bei der alkalischen Verseifung führen Verunreinigungen im Nitril der Formel (II) zu Nebenprodukten in der erhaltenen Säure der Formel (I). Bei solchen Verunreinigungen im Nitril handelt es sich entsprechend dem Herstellungsweg hauptsächlich um Schwefelverbindungen und elementaren Schwefel. Bei den aus diesen enstehenden Nebenprodukten handelt es sich in der Hauptsache um das Disulfid der Formel (III).

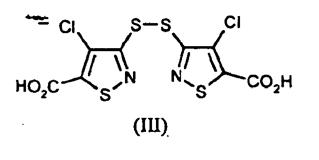

Die Bildung der Nebenkomponente der Formel (III) lässt sich weitgehend unterdrücken, indem die alkalische Verseifung des Nitrils in hoher Verdünnung durchgeführt wird. Daraus resultieren jedoch geringe Raum-Zeit-Ausbeuten, was für einen ökonomischen Prozess im technischen Maßstab sehr nachteilig ist.

Eine andere Möglichkeit zur Vermeidung schwefelhaltiger Nebenprodukte in der Säure der Formel (I) besteht darin, das Nitril der Formel (II) frei von schwefelhaltigen Verunreinigungen zu erzeugen und in dieser sauberen Form in die Verseifung einzusetzen. Dies ist jedoch äußerst schwierig und aufwendig. So wird beispielsweise entsprechend US 3,341,547 und US 3,393,992 das Reaktionsgemisch nach dem ersten Schritt des Herstellungsverfahrens über ein Filtrierhilfsmittel filtriert. Anschließend wird das Filtrat mit sehr viel Wasser versetzt. Der so erhaltene Feststoff wird abgesaugt und mit weiterem Wasser gewaschen. Dadurch resultieren große Mengen mit organischen Verbindungen belasteten und deshalb nur schwierig zu entsorgendem Abwassers. Das so erhaltene Rohprodukt muss nun in zusätzlichen Schritten weiter gereinigt werden. Dazu kann entsprechend US 3,341,547 und US 3,393,992 das Nitril einer Wasserdampfdestillation unterzogen oder aus Cyclohexan umkristallisiert werden.

Eine andere Möglichkeit besteht darin, das Rohprodukt mit einem Lösungsmittel wie beispielsweise Methylenchlorid oder Toluol zu extrahieren. Aber auch das so erhaltenen Nitril der Formel (I) enthält in der Regel noch bedeutende Mengen an Schwefel und/oder Schwefelverbindungen.

Grundsätzlich ist es auch möglich, das Nitril durch Destillation zu reinigen. Dies ist jedoch mit hohen Ausbeuteverlusten verbunden. Außerdem ergeben sich technische Probleme, da die Verbindung leicht sublimiert.

Aus US 5,240,951 ist bekannt geworden, nach dem ersten Schritt des Herstellungsverfahrens zu dem Reaktionsgemisch Wasser zu geben und den so erhaltenen Feststoff abzusaugen. Der isolierte Feststoff wird dann in Essigsäureethylester aufgenommen. Unlösliche Anteile müssen wiederum abgesaugt, das Filtrat getrocknet und anschließend eingeengt werden. Diese Schritte sind alle zeit-, energie- und arbeitsaufwendig.

Andere bekannt gewordene Methoden zur Synthese des Nitrils der Formel (II) sind die Umsetzung von Teträchlor-bernsteinsäuredinitril bzw. Trichloracetonitril mit elementarem Schwefel bei hohen Temperaturen (DE 2 231 097 und DE 2 231 098). Aber auch diese Verfahren ergeben ein stark mit Schwefel und -verbindungen verunreinigtes Nitril der Formel (I), so dass hier ebenfalls aufwendige Reinigungsoperationen notwendig sind.

Die Verwendung von 3,4-Dichlor-isothiazol-carbonsäure der Formel (I) liegt zum einen in ihrer Wirkung als Pflanzenwuchsregulator (siehe beispielsweise US 3,341,547 und US 3,393,992). Zum anderen ist sie ein wichtiges Zwischenprodukt zur Herstellung fungizider Wirkstoffe (s. beispielsweise WO 99/24413 und WO 2004/002968).

Somit besteht weiterhin Bedarf an einem einfachen, technisch vorteilhaft durchführbaren Verfahren zur Herstellung von 3,4-Dichlor-isothiazol-carbonitril der Formel (II) und Verseifung dieses Nitrils zur Säure der Formel (I).

Es wurde nun gefunden, dass sich 3,4-Dichlor-isothiazol-carbonsäure der Formel (I) überraschend einfach in guter Reinheit erhalten lässt, indem man nach dem ersten Schritt des Herstellverfahrens das Lösungsmittel abdestilliert, den Rückstand in einem Alkohol aufnimmt, zur Abtrennung und Entfernung von Natriumchlorid und Schwefel filtriert, und in der alkoholischen Lösung im zweiten Schritt des Herstellungsverfahrens alkalisch verseift.

Gegenstand der Erfindung ist dementsprechend ein Verfahren zur Herstellung von 3,4-Dichlor-isothiazol-carbonsäure der Formel (I), dadurch gekennzeichnet, dass man im ersten Schritt des Verfahrens ein Cyanid, Schwefelkohlenstoff und Chlor zum 3,4-Dichlor-isothiazol-carbonitril der Formel (II) umsetzt, dieses nach Abdestillation des Lösungsmittels, Versetzen mit einem Alkohol und Filtration als Lösung in dem Alkohol isoliert und anschließend im zweiten Schritt des Verfahrens in diesem Alkohol alkalisch verseift.

Das erfindungsgemäße Verfahren vermeidet die wässrige Aufarbeitung des Nitrils und somit großen Mengen eines stark mit organischen Substanzen belasteten Abwassers. Im ersten Schritt des erfindungsgemäßen Verfahrens wird nur ein Filtrationsschritt benötigt. Aufwendige Reinigungsoperationen für das Nitril können entfallen.

Es kann als sehr überraschend bezeichnet werden, dass die Säure der Formel (I) auf diese einfache Weise in ausreichender Reinheit isoliert wird.

Ebenfalls überraschend ist, dass die Abtrennung des Schwefels durch Filtration nach Versetzen mit einem Alkohol in einfacher Weise sehr gut möglich ist.

Der erste Verfahrensschritt wird vorzugsweise so gestaltet, dass man ein Cyanid in einem Lösungsmittel vorlegt, mit CS₂ versetzt, vorzugsweise durch Zutropfen, und nach erfolgter Umsetzung vorzugsweise gasförmiges Chlor einleitet.

Als Cyanid eignen sich vorzugsweise anorganische Cyanide, wie Natriumcyanid und Kaliumcyanid.
Besonders bevorzugt ist Natriumcyanid.

Die Umsetzung des Cyanids mit Schwefelkohlenstoff im ersten Schritt des Verfahrens erfolgt in bekannter Weise bei Temperaturen zwischen vorzugsweise 30 und 80°C.

Die Reaktionszeit für die Umsetzung von Cyanid mit Schwefelkohlenstoff im ersten Schritt des Verfahrens beträgt typischerweise 0,5 bis 6 Stunden, bevorzugt 2 bis 4 h.

Die Chlorierung im ersten Schritt des erfindungsgemäßen Verfahrens erfolgt im allgemeinen bei Temperaturen zwischen 10 und 80 °C. Bevorzugt sind Temperaturen zwischen 20 und 60°C.

Die Reaktionszeit für die Chlorierung im ersten Schritt des erfindungsgemäßen Verfahrens liegt im allgemeinen zwischen 1 und 6 Stunden und hängt von der Temperatur bei der Chlorierung ab. Bevorzugt führt man die Einleitung des Chlors bei 30°C innerhalb von 2 Stunden durch, und lässt dann 1 bis 5 Stunden bei 20°C, oder 1 bis 2 Stunden bei 20°C und 1 bis 2 Stunden bei 60°C abreagieren.

Das Molverhältnis von Cyanid und CS₂ liegt im allgemeinen zwischen 1,5 : 1 und 1 : 1,5, vorzugsweise zwischen 1,3 : 1 und 1 : 1,3, insbesondere beträgt es etwa 1:1.

Die Chlormenge im ersten Schritt des erfindungsgemäßen Verfahrens liegt im allgemeinen zwischen 0,5 und 1,5 Mol pro Mol Natriumcyanid. Bevorzugt sind 1 bis 1,3 Mol Chlor pro Mol Natriumcyanid.

Nicht abreagierte überschüssige Mengen an Chlor werden vorzugsweise bei Temperaturen zwischen 20 und 60°C durch Einleiten eines Inertgases, wie Stickstoff oder Argon, entfernt. Als Lösungsmittel im ersten Schritt des Verfahrens werden vorzugsweise Amide, wie Formamid, N,N-Dimethylformamid, N,N-Dimethylacetamid oder N-Methyl-pyrrolidon, verwendet. Besonders bevorzugt sind N,N-Dimethyl-formamid und N,N-Dimethylacetamid.

Das Lösungsmittel aus dem ersten Schritt des Verfahrens kann bei Normaldruck oder reduziertem Druck abdestilliert werden. Zur Vermeidung höherer Temperaturen wird man in der Regel bei vermindertem Druck destillieren. Bei dieser Destillation werden gleichzeitig Schwefelchloride mit abdestilliert. Je nach Siedepunkt des verwendeten Lösungsmittels wird man dieses ganz oder weitgehend frei von solchen Schwefelchloriden erhalten. Zur besseren Abtrennung der Schwefelchloride kann gegebenenfalls über eine Kolonne destilliert werden. Dies kann direkt aus dem Reaktionsgemisch erfolgen, oder aber in einer zweiten, nachgeschalteten fraktionierten Destillation. Vorzugsweise wird nur soweit abdestilliert, dass noch ein rührfähiger Rückstand verbleibt.

Die Zugabe des Alkohols zur Isolierung des Nitrils als alkoholische Lösung erfolgt im allgemeinen nach dem Abdestillieren des Lösungsmittels. Es können ein oder mehrwertige Alkohole mit einer Kettenlänge von vorzugsweise 1 bis 6 Kohlenstoffatomen; gegebenenfalls auch in Mischung, verwendet werden.

Bevorzugt sind einwertige C₁- bis C₆-Alkohole; besonders bevorzugt sind Methanol und Ethanol.

Die Isolierung des Nitrils als Lösung im ersten Schritt des Verfahrens erfolgt im allgemeinen bei Temperaturen von 20 bis 100°C; bevorzugt bei 40 bis 80°C.

Die Menge an Alkohol ist üblicherweise so bemessen, dass sie ausreicht, die erwartete Menge an Nitril bei der Temperatur der Isolierung zu lösen.

Zur alkalischen Verseifung im zweiten Schritt des Verfahrens wird die Lösung des Nitrils im verwendeten Alkohol im allgemeinen mit einer Lauge, wie Natron- oder Kalilauge, versetzt und erhitzt. Anschließend wird der Alkohol abdestilliert, der wässrige Rückstand mit einer Säure, beispielsweise Salz- oder Schwefelsäure, sauer gestellt und die ausgefallene 3,4-Dichlor-isothiazol-carbonsäure der Formel (I) abgesaugt und getrocknet.

Die Menge an Lauge im zweiten Schritt des Verfahrens beträgt typischerweise 1,5 bis 5 Mol pro Mol Nitril. Bevorzugt sind 2 bis 3,5 Mol pro Mol Nitril.

Die Temperatur bei der Verseifung beträgt im allgemeinen 20 bis 60°C, bevorzugt 30 bis 45°C.

Die Dauer der Verseifung im zweiten Schritt des erfindungsgemäßen Verfahrens beträgt typischerweise 2 bis 8 Stunden.

Die erfindungsgemäß hergestellte Säure (I) findet beispielsweise wie oben angegeben Verwendung als Pflanzenwuchsregulator und als Zwischenprodukt zur Herstellung agrochemischer Wirkstoffe, insbesondere Fungizide.

Die Erfindung wird im folgenden anhand von Beispielen näher beschrieben.

### Beispiel 1

### a) Herstellung von 3,4-Dichlor-isothiazol-carbonitril

Zu 51,6 g [1 Mol] NaCN (95%ig) in 428 g DMF wurden innerhalb 1 Stunde bei 30-50°C (leicht exotherm) 76,5 g [Mol] CS₂ (99,5%ig) getropft. Man rührte 2 Stunden bei 60°C und ließ dann auf ca. 30°C abkühlen. Unter Eiskühlung wurden bei etwa 30°C innerhalb von 2 Stunden 79,4 g [1,12 Mol] Chlor eingeleitet. Man rührte anschließend noch 3 Stunden bei 60°C. Überschüssiges Chlor wurde während 1 Stunde bei Raumtemperatur mit Stickstoff ausgetrieben. Dann wurden bei ca. 32 mbar alle bis 100°C Badtemperatur flüchtigen Anteile abdestilliert (406,5 g Destillat). Der Rückstand wurde mit 250 ml Methanol kurz aufgekocht und die erhaltene Suspension noch warm filtriert. Der Filterrückstand wurde mit 150 ml warmen MeOH gewaschen und getrocknet (ergab 107,4 g Feststoff, der aus NaCl und Schwefel bestand). Das Filtrat wurde am Rotationsverdampfer etwas aufkonzentriert. Man erhielt 250,3 g einer dunkelbraunen Lösung, die 18,1% (w/w) Nitril enthielt. Daraus ergibt sich eine Ausbeute von 50,6% d. Theorie.

### b) Herstellung von 3,4-Dichlor-isothiazol-carbonsäure

53,33 g 45%ige Natronlauge wurden vorgelegt und mit 220 ml Wasser verdünnt. Unter Kühlung wurde dann die 250 g Lösung bzw. Suspension aus Schritt a) zudosiert. Da nach 6 Stunden bei 40°C noch Nitril vorhanden war, wurden weitere 0,1 Mol NaOH zugesetzt und nochmals 2 Stunden bei 40°C gerührt. Das MeOH wurde anschließend am Rotationsverdampfer weitgehend abgezogen. Die verbliebene Lösung wurde über etwas Kieselgur filtriert und unter Eiskühlung mit konz. Salzsäure sauer gestellt. Der ausgefallene Feststoff wurde abgesaugt, mit Wasser gewaschen und getrocknet. Auf diese Weise resultierten 60,8 g beiger Feststoff. Analytik gegen Standard: 74,2% (w/w). Damit ergibt sich eine Ausbeute von 90,1% d.Theorie. Das Disulfid der Formel (III) war nach GC zu nur 0,3% enthalten.

### Beispiel 2

### a) Herstellung von 3,4-Dichlor-isothiazol-carbonitril

Zu 51,6 g [1 Mol] NaCN (95%ig) in 428 g DMF wurden innerhalb 1 Stunde bei 30-50°C (leicht exotherm) 76,5 g [1 Mol] CS₂ (99,5%ig) getropft. Man rührte 2 Stunden bei 60°C und ließ dann auf ca. 30°C abkühlen. Unter Eiskühlung wurden bei etwa 30°C innerhalb von 2 Stunden 79,4 g [1,12 Mol] Chlor eingeleitet. Man rührte anschließend noch 1 Stunde bei Raumtemperatur. Überschüssiges Chlor wurde innerhalb 1 Stunde bei Raumtemperatur mit Stickstoff ausgetrieben. Dann wurden bei ca. 20 mbar alle bis 100°C Badtemperatur flüchtigen Anteile abdestilliert (421,7 g gelbliches Destillat). Der noch warme Destillationssumpf wurde mit 250 ml Methanol kurz aufgekocht und die erhaltene Suspension noch warm filtriert. Der Filterrückstand wurde mit 100 ml warmen MeOH gewaschen und getrocknet (ergab 108,3 g Feststoff, der aus NaCl und Schwefel bestand). Das Filtrat wurde am Rotationsverdampfer etwas aufkonzentriert. Man erhielt 205 g einer dunkelbraunen Lösung, die 21% (w/w) Nitril enthielt. Daraus ergibt sich eine Ausbeute von 48,1% d. Theorie.

### b) Herstellung von 3,4-Dichlor-isothiazol-carbonsäure

44,45 g 45%ige Natronlauge wurden vorgelegt und mit 220 ml Wasser verdünnt. Unter Kühlung wurde dann die 205 g Lösung bzw. Suspension aus Schritt a) zudosiert. Da nach 6 Stunden bei 40°C noch Nitril vorhanden war, wurden weitere 8,9 g 45%ige Natronlauge zugesetzt und nochmals 2 Stunden bei 40°C gerührt. Das MeOH wurde anschließend am Rotationsverdampfer weitgehend abgezogen. Die verbliebene Lösung wurde über etwas Kieselgur filtriert und unter Eiskühlung mit konz. Salzsäure sauer gestellt. Der ausgefallene Feststoff wurde abgesaugt, mit Wasser gewaschen und getrocknet. Auf diese Weise resultierten 52,4 g beiger Feststoff.
HPLC: 79,1 F1.-%. Analytik gegen Standard: 75,5% (w/w). Damit ergibt sich eine Ausbeute von 83,1% d.Theorie.
Das Disulfid der Formel (III) wurde nicht gefunden.

### Beispiel 3

### a) Herstellung von 3,4-Dichlor-isothiazol-carbonitril

Zu 51,6 g [1 Mol] NaCN (95%ig) in 428 g DMF wurden innerhalb 1 Stunde bei 30-50°C (leicht exotherm) 76,5 g [1 Mol] CS₂ (99,5%ig) getropft. Man rührte 2 Stunden bei 60°C und ließ dann auf ca. 30°C abkühlen. Unter Eiskühlung wurden bei etwa 30°C innerhalb von 2 Stunden 79,4 g [1,12 Mol] Chlor eingeleitet. Man rührte anschließend noch 3 Stunden bei Raumtemperatur.
Überschüssiges Chlor wurde innerhalb 1 Stunde bei Raumtemperatur und 1 Stunde bei 60°C mit Argon ausgetrieben. Dann wurden bei ca. 20 mbar alle bis 100°C Badtemperatur flüchtigen Anteile abdestilliert (426,7 g gelbliches Destillat). Der noch warme Destillationssumpf wurde mit 250 ml Methanol kurz aufgekocht und die erhaltene Suspension noch warm filtriert. Der Filterrückstand wurde mit 100 ml warmen MeOH gewaschen und getrocknet (ergab 105,5 g Feststoff, der aus NaCl und Schwefel bestand). Das Filtrat wurde am Rotationsverdampfer etwas aufkonzentriert. Man erhielt 210,3 g einer dunkelbraunen Lösung, die 24,9% (w/w) Nitril enthielt. Daraus ergibt sich eine Ausbeute von 58,5% d. Theorie.

### b) Herstellung von 3,4-Dichlor-isothiazol-carbonsäure

62,2 g 45%ige Natronlauge wurden vorgelegt und mit 220 ml Wasser verdünnt. Unter Kühlung wurde dann die 210 g Lösung bzw. Suspension aus Schritt a) zudosiert. Nach 6 Stunden bei 40°C wurde das MeOH anschließend am Rotationsverdampfer weitgehend abgezogen. Die verbliebene Lösung wurde über etwas Kieselgur filtriert und unter Eiskühlung mit konz. Salzsäure sauer gestellt. Der ausgefallene Feststoff wurde abgesaugt, mit Wasser gewaschen und getrocknet. Auf diese Weise resultierten 51,2 g beiger Feststoff. Analytik gegen Standard: 83, 1 % (w/w). Damit ergibt sich eine Ausbeute von 78, 1 % d.Theorie.
Das Disulfid der Formel (III) wurde nicht gefunden.

### Beispiel 4

### a) Herstellung von 3,4-Dichlor-isothiazol-carbonitril

Zu 51,6 g [1 Mol] NaCN (95%ig) in 428 g DMF wurden innerhalb 1 Stunde bei 30-50°C (leicht exotherm) 76,5 g [Mol] CS₂ (99,5%ig) getropft. Man rührte 2 Stunden bei 60°C und ließ dann auf ca. 30°C abkühlen. Unter Eiskühlung wurden bei etwa 30°C innerhalb von 2 Stunden 79,4 g [1,12 Mol] Chlor eingeleitet. Man rührte anschließend noch 1 Stunde bei Raumtemperatur. Dann wurden bei ca. 20 mbar alle bis 100°C Badtemperatur flüchtigen Anteile abdestilliert (425 g gelbliches Destillat). Der noch warme Destillationssumpf wurde mit 250 ml Ethanol kurz aufgekocht und die erhaltene Suspension noch warm filtriert. Der Filterrückstand wurde mit 100 ml warmen EtOH gewaschen und getrocknet (ergab 109,35 g Feststoff, der aus NaCl und Schwefel bestand). Das Filtrat wurde am Rotationsverdampfer etwas aufkonzentriert. Man erhielt 159,5 g einer dunkelbraunen Lösung, die 27,1% (w/w) Nitril enthielt. Daraus ergibt sich eine Ausbeute von 48,3% d. Theorie.

### b) Herstellung von 3,4-Dichlor-isothiazol-carbonsäure

53,3 g 45%ige Natronlauge wurden vorgelegt und mit 220 ml Wasser verdünnt. Unter Kühlung wurde dann die 159,5 g Lösung bzw. Suspension aus Schritt a) zudosiert. Da nach 6 Stunden bei 40°C noch Nitril vorhanden war, wurden weitere 13.3 g 45%ige Natronlauge zugesetzt und nochmals 2 Stunden bei 40°C gerührt. Danach wurde das Ethanol am Rotationsverdampfer weitgehend abgezogen. Die verbliebene Lösung wurde über etwas Kieselgur filtriert und unter Eiskühlung mit konz. Salzsäure sauer gestellt. Der ausgefallene Feststoff wurde abgesaugt, mit Wasser gewaschen und getrocknet. Auf diese Weise resultierten 65,6 g beiger Feststoff.
Analytik gegen Standard: 81,2% (w/w). Damit ergibt sich eine Ausbeute von 86,3% d.Theorie.
Das Disulfid der Formel (III) wurde zu 1,9% nachgewiesen.

### Beispiel 5

### a) Herstellung von 3,4-Dichlor-isothiazol-carbonitril

Zu 51,6 g [1 Mol] NaCN (95%ig) in 428 g DMF wurden innerhalb 1 Stunde bei 30-50°C (leicht exotherm) 76,5 g [1 Mol] CS₂ (99,5%ig) getropft. Man rührte 2 Stunden bei 60°C und ließ dann auf ca. 30°C abkühlen. Unter Eiskühlung wurden bei etwa 30°C innerhalb von 2 Stunden 79,4 g [1,12 Mol] Chlor eingeleitet. Man rührte anschließend noch 1 Stunde bei Raumtemperatur. Dann wurden die im Siedebereich 50-55°C/20 mbar flüchtigen Anteile abdestilliert (433 g gelbliches Destillat). Der noch warme Destillationssumpf wurde mit 250 g Isopropanol kurz aufgekocht und die erhaltene Suspension noch warm filtriert. Der Filterrückstand wurde mit 150 ml warmen Isopropanol gewaschen und getrocknet (ergab 104,5 g Feststoff, der aus NaCl und Schwefel bestand). Das Filtrat wurde am Rotationsverdampfer etwas aufkonzentriert. Man erhielt 267,6 g einer dunkelbraunen Lösung, die 14,85% (w/w) Nitril enthielt. Daraus ergibt sich eine Ausbeute von 44,2% d. Theorie.

### b) Herstellung von 3,4-Dichlor-isothiazol-carbonsäure

66,6 g 45%ige Natronlauge wurden vorgelegt und mit 220 ml Wasser verdünnt. Unter Kühlung wurde dann die 267 g Lösung bzw. Suspension aus Schritt a) zudosiert. Nach 8 Stunden bei 40°C wurde das Isopropanol am Rotationsverdampfer teilweise abgezogen. Die verbliebene Lösung wurde über etwas Kieselgur filtriert und unter Eiskühlung mit konz. Salzsäure sauer gestellt. Der ausgefallene Feststoff wurde abgesaugt, mit Wasser gewaschen und getrocknet. Es resultierte eine Ausbeute von 90%.
Das Disulfid der Formel (III) wurde zu 4% nachgewiesen.

### Beispiel 6

Aus 2 Ansätzen analog Beispiel 2, Stufe a) wurden in Summe 850 g Rohdestillat erhalten. Dieses wurde dann an einer 30 cm-Füllkorperkolonne im Vakuum fraktioniert. Dabei wurden 392 g (= 46%) einer DMF-Fraktion mit dem Siedepunkt 60°C bei 40-42 mbar erhalten, die einen Schwefelgehalt von < 0, 1 % aufwies.

### Vergleichsbeispiel

### a) Herstellung von 3,4-Dichlor-isothiazol-carbonitril

Zu 49 g [1 Mol] NaCN in 500 ml DMF wurden innerhalb 1 Stunde bei 30-50°C (leicht exotherm) 76,1 g [1 Mol] CS₂ getropft. Man rührte 1 Stunde bei 60°C und ließ dann über Nacht bei Raumtemperatur stehen. Man erhielt eine Suspension feiner rot-brauner Kristalle. Unter Eiskühlung wurden bei Raumtemperatur 78 g [1,1 Mol] Chlor eingeleitet. Man rührte über Nacht bei Raumtemperatur und dann noch 3 Stunden bei 60°C.
Ggf. noch vorhandenes überschüssiges Chlor wurde bei Raumtemperatur mit Stickstoff ausgetrieben. Das Reaktionsgemisch wurde dann unter Rühren in 1,5 Liter Eiswasser gegeben. Es wurde 30 Minuten bei 0-5°C gerührt und der erhaltene Feststoff dann abgesaugt. Nach Waschen mit 300 ml Wasser und Trocknen verblieben 110 g brauner Feststoff, der nach HPLC 68,9 Fl.-% Zielprodukt und 4,7 Fl.-% Schwefel enthielt. Bei dieser Analytik werden viele Verunreinigungen und der Schwefel allerdings nicht richtig erfasst, wie die Gehaltsbestimmung gegen einen analytischen Standard zeigt: 39,5%(w/w) (entspricht 48,6% Ausbeute).
Bei dieser Art der Aufarbeitung fallen mehr als 2000 g eines mit DMF und anderen Stoffen stark belasteten Abwassers an, welches entsorgt werden muss.

### b) Herstellung von 3,4-Dichlor-isothiazol-carbonsäure

10,2 g 45%ige Natronlauge (entsprechend 115 mmol NaOH) wurden mit 84 ml Wasser verdünnt. Unter Kühlung gab man bei max. 40°C 22,66 g Nitril aus Stufe a) (50 mmol) hinzu. Das Reaktionsgemisch wurde 3 Stunden bei 40°C gerührt. Man filtrierte über etwas Celite, wusch mit 50 ml Wasser nach und stellte das Filtrat unter Eiskühlung mit konz. Salzsäure auf pH 1-2. Nach 30 Minuten wurde der ausgefallene Feststoff abgesaugt, mit zweimal 25 ml Wasser gewaschen und getrocknet. Man erhielt 8,45 g beigen Feststoff.
HPLC: 90,9 Fl.-% Zielprodukt (77,6% Ausbeute); 1,4 Fl.-% Disulfid; 4,4 Fl.-% Schwefel.

## Patentansprüche

1. Verfahren zur Herstellung von 3,4-Dichlor-isothiazol-carbonsäure der Formel (I), 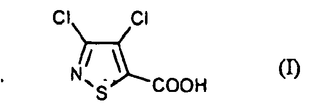 **dadurch gekennzeichnet, dass** man im ersten Schritt des Verfahrens ein Cyanid, Schwefelkohlenstoff und Chlor zum 3,4-Dichlor-isothiazol-carbonitril der Formel 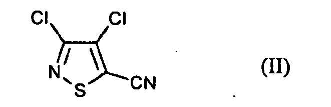 umsetzt, dieses nach Abdestillation des Lösungsmittels, Versetzen mit einem Alkohol und Filtration als Lösung in dem Alkohol isoliert, und anschließend im zweiten Schritt des Verfahrens in diesem Alkohol alkalisch zur Säure der Formel (I) verseift.

2. Verfahren gemäß Anspruch 1, **dadurch gekennzeichnet, dass** als Alkohol ein ein- oder mehrwertiger C₁- bis C₆-Alkohol verwendet wird.

3. Verfahren gemäß Anspruch 1 oder 2 **dadurch gekennzeichnet, dass** als Alkohol Methanol oder Ethanol verwendet wird.

4. Verfahren nach einem oder mehreren der vorhergehenden Ansprüche, **dadurch gekennzeichnet, dass** als Alkohol Methanol verwendet wird.

5. Verfahren gemäß einem oder mehreren der vorhergehenden Ansprüche, **dadurch gekennzeichnet, dass** als Lösungsmittel im ersten Schritt des Verfahrens Dimethylformamid oder Dimethylacetamid verwendet wird.

6. Verfahren gemäß einem oder mehreren der vorhergehenden Ansprüche, **dadurch gekennzeichnet, dass** die Chlorierung im ersten Schritt des Verfahrens bei Temperaturen zwischen 20 und 60°C durchgeführt wird.

7. Verfahren gemäß einem oder mehreren der vorhergehenden Ansprüche, **dadurch gekennzeichnet, dass** im ersten Schritt des Verfahrens Reaktionszeit und -temperatur nach Beendigung der Chloreinleitung bei 1 bis 5 Stunden bei 20°C, oder 1 bis 2 Stunden bei 20°C und 1 bis 2 Stunden bei 60°C liegen.

8. Verfahren gemäß einem oder mehreren der vorhergehenden Ansprüche, **dadurch gekennzeichnet, dass** im ersten Schritt des Verfahrens nach Beendigung der Chlorierung überschüssiges Chlor bei Temperaturen zwischen 20 und 60°C mittels eines Inertgases entfernt wird.

9. Verfahren gemäß einem oder mehreren der vorhergehenden Ansprüche, **dadurch gekennzeichnet, dass** als Cyanid Natriumcyanid eingesetzt wird.

10. Verfahren gemäß einem oder mehreren der vorhergehenden Ansprüche, **dadurch gekennzeichnet, dass** zur alkalischen Verseifung Natron- oder Kalilauge verwendet wird.

## Claims

1. Process for preparing 3,4-dichloroisothiazolecarboxylic acid of the formula (I), 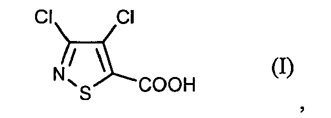 **characterized in that** a cyanide, carbon disulphide and chlorine are reacted in the first step of the process to give 3,4-dichloroisothiazolecarbonitrile of the formula 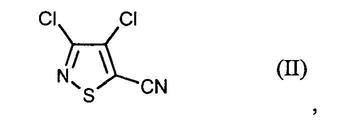 this is isolated as a solution in the alcohol after distilling off the solvent, admixing with an alcohol and filtering, and then, in the second step of the process, hydrolysed under alkaline conditions in this alcohol to give the acid of the formula (I).

2. Process according to Claim 1, **characterized in that** the alcohol used is a mono- or polyhydric C₁- to C₆ alcohol.

3. Process according to Claim 1 or 2, **characterized in that** the alcohol used is methanol or ethanol.

4. Process according to one or more of the preceding claims, **characterized in that** the alcohol used is methanol.

5. Process according to one or more of the preceding claims, **characterized in that** the solvent used in the first step of the process is dimethylformamide or dimethylacetamide.

6. Process according to one or more of the preceding claims, **characterized in that** the chlorination in the first step of the process is carried out at temperatures between 20 and 60°C.

7. Process according to one or more of the preceding claims, **characterized in that**, in the first step of the process, reaction time and temperature after the chlorine introduction has ended, are 1 to 5 hours at 20°C, or 1 to 2 hours at 20°C and 1 to 2 hours at 60°C.

8. Process according to one or more of the preceding claims, **characterized in that**, in the first step of the process, after the chlorination has ended, excess chlorine is removed by means of an inert gas at temperatures between 20 and 60°C.

9. Process according to one or more of the preceding claims, **characterized in that** the cyanide used is sodium cyanide.

10. Process according to one or more of the preceding claims, **characterized in that** sodium hydroxide solution or potassium hydroxide solution is used for the alkaline hydrolysis.

## Revendications

1. Procédé de production d'acide 3,4-dichlorisothiazole-carboxylique de formule (I) 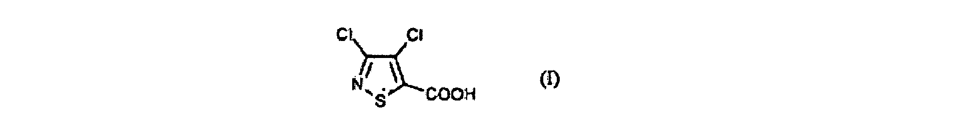 **caractérisé en ce que**, dans la première étape du procédé, on fait réagir un cyanure, du sulfure de carbone et du chlore pour former le 3,4-dichlorisothiazole-carbonitrile de formule 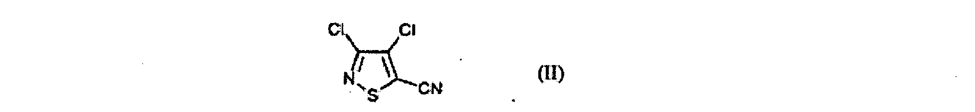 qu'on isole, après avoir chassé le solvant par distillation, ajouté un alcool et filtré, sous forme de solution dans l'alcool, après quoi, dans la seconde étape du procédé, on le saponifie dans cet alcool par voie alcaline en l'acide de formule (I).

2. Procédé suivant la revendication 1, **caractérisé en ce qu'**on utilise comme alcool un alcool en C₁ à C₆ monovalent ou polyvalent.

3. Procédé suivant la revendication 1 ou 2,
**caractérisé en ce qu'**on utilise comme alcool du méthanol ou de l'éthanol.

4. Procédé suivant une ou plusieurs des revendications précédentes, **caractérisé en ce qu'**on utilise comme alcool du méthanol.

5. Procédé suivant une ou plusieurs des revendications précédentes, **caractérisé en ce qu'**on utilise comme solvant, dans la première étape du procédé, du diméthylformamide ou du diméthylacétamide.

6. Procédé suivant une ou plusieurs des revendications précédentes, **caractérisé en ce que** la chloration dans la première étape du procédé est conduite à des températures comprises entre 20 et 60°C.

7. Procédé suivant une ou plusieurs des revendications précédentes, **caractérisé en ce que**, dans la première étape du procédé, le temps et la température de réaction, après la fin de l'introduction de chlore, sont de 1 à 5 heures à 20°C, ou bien de 1 à 2 heures à 20°C et 1 à 2 heures à 60°C.

8. Procédé suivant une ou plusieurs des revendications précédentes, **caractérisé en ce que** dans la première étape du procédé, après la fin de la chloration, le chlore en excès est chassé à des températures comprises entre 20 et 60°C au moyen d'un gaz inerte.

9. Procédé suivant une ou plusieurs des revendications précédentes, **caractérisé en ce qu'**on utilise comme cyanure du cyanure de sodium.

10. Procédé suivant une ou plusieurs des revendications précédentes, **caractérisé en ce qu'**on utilise pour la saponification alcaline de la lessive de soude ou de la lessive de potasse.
